# EUROPEAN PATENT SPECIFICATION

(11) **EP 1 045 656 B1**
(45) Date of publication and mention of the grant of the patent: **29.01.2003**
(21) Application number: 98952856.7
(22) Date of filing: 09.11.1998
(51) Int. Cl.: A45D 40/00

(54) **SEAL FOR A DEVICE COMPRISING AN ENVELOPE CONTAINING A FLUID MATERIAL**
ABDICHTUNG EINES GERÄTES MIT EINER FLÜSSIGKEIT BEINHALTENDE HÜLLE
JOINT POUR UN DISPOSITIF COMPOSE D'UNE ENVELOPPE CONTENANT UN MATERIAU LIQUIDE

(30) Priority: 07.11.1997 GB 9723451
(43) Date of publication of application: 25.10.2000
(73) Proprietor: AKI INC., New York, NY 10022 (US)
(72) Inventor: BISHOPP, Derek, Albert, Northampton NN5 5LM (GB)
(74) Representative: Dummett, Thomas Ian Peter
(86) International application number: GB9803313
(87) International publication number: WO99023908

(56) References cited:
- GB-A- 2 284 155
- US-A- 4 376 483
- US-A- 5 439 172

## Description

The present invention relates to a device, notably to a device for sampling fragrances or other volatile materials.

### BACKGROUND TO THE INVENTION:

In our US Patents No 5,341,992 and 5,439,172, which discloses the features of the preamble of claim 1, we have described various forms of sampler for a fluid volatile material, for example a liquid fragrance oil in an alcohol or other fluid carrier or diluent. In a preferred embodiment, the sampler comprises a base layer, notably one made from a polymer, especially a polyester polymer, which is micro-permeable to an organic volatile fluid applied to it, whereby the base layer absorbs the fluid on a molecular scale and acts as a reservoir for the fluid. A cover layer, preferably also of a polyester or similar polymer, is applied over at least that area of the base layer which carries the volatile fluid. The cover is secured in position, for example by an adhesive, preferably in the form of an annulus around the area of the base layer which carries the volatile fluid, so that the base and cover layers form, with the adhesive seal therebetween, a substantially vapour proof envelope for the volatile material. When the cover layer is peeled back or otherwise removed to expose that part of the base layer carrying the volatile fluid to the atmosphere, the volatile fluid can volatilise directly into the atmosphere or the surrounds of the device.

The adhesive is preferably of a pressure sensitive type, for example a water based acrylic adhesive, so that the seal between the base and cover layers can be broken when the cover layer is peeled back, and yet the cover layer can be re-applied to the base layer to re-form the seal and prevent further release of the volatile material from the base layer once a user has smelt the vapours released from the base layer.

Such devices provide a simple means by which a volatile material can be presented to a user in a substantially sealed sampler from which little or none of the volatile material escapes until required, and which can then be resealed to prevent further escape once the user has sampled the volatile material within the device. However, we have found that with prolonged storage of such devices where the volatile liquid material is a fragrance oil or the like, the apparent strength of the fragrance perceived by a user when the cover layer is peeled back from the base layer is less than would have been expected. Attempts to apply a greater loading of the volatile fluid to the base layer, for example by increasing the nip gap of the printing roller used to apply the fluid composition containing the liquid volatile material as described in our US Patent 5,439,172, have not been wholly successful. Loss of perceived strength of the fragrance with time remains a problem for some types of fragrance.

Since the base and polymer layers are micro-permeable to the liquid volatile material, one possible route for the loss of volatile liquid could be the permeation of the liquid through the base and/or cover layers. However, we have found that the amount of liquid permeating through the base and cover layers is insufficient to account for the perceived loss.

The fluid composition containing the liquid volatile material is usually applied to form a substantially circular area of material on the base layer. Such application is preferably done using by a conventional printing roller application technique which applies only about 3 to 30 micrograms of the fluid composition per square metre of the base layer. A continuous or discontinuous ring of adhesive is applied radially outwardly of the spot so as to leave an annular gap of from 1 to 5 mms between the outer edge of the spot and the inner rim of the ring of adhesive. This gap is provided to minimise contact between the adhesive composition and the liquid fragrance or other liquid volatile material whose odour (or rendition) could be adversely affected by an interaction with the polymers, solvents or other components of the adhesive. The gap closes up due to pressure applied to the device during subsequent stages of its manufacture and storage, which is to be expected, and has been allowed for in selecting the width of the gap to be left initially.

Surprisingly, we have found that the liquid volatile material also migrates laterally within the polymer structure. Such molecular scale migration is not perceptible to the human eye, and we believe that once the liquid contacts the adhesive, breakdown of the integrity of the adhesive seal is more rapid and far greater than would have been expected. As a result, it is the imperceptible lateral migration of the liquid volatile material through the degraded adhesive seal which is the prime cause of the subsequent perceived loss of volatile material from the base layer.

The main purpose for using a pressure sensitive adhesive to form the seal between the base and cover layers is to enable the sampler to be re-sealed once opened. A conventional heat seal would not achieve this, since it has to be ruptured to separate the sealed components and cannot be re-formed once ruptured. Surprisingly, we have now found that a heat seal can be used in conjunction with an adhesive seal to reduce the above problems. A heat seal is formed by fusing the base and cover layers together and therefore prima facie consists of the same materials as the remainder of the base and cover layers. It would therefore have been expected that if migration of the volatile material through the molecular structure of the polymer of either or both of the cover and base layers was occurring, mere fusing of one layer to the other would not significantly reduce the rate of migration and hence the loss of perceived strength of the fragrance. However, we have found that this is not the case and that the use of a heat seal in addition to the adhesive seal reduces the apparent loss of fragrance strength. The use of the adhesive ring in addition to the heat seal enables the cover layer to be resealed to the base layer once the heat seal has been ruptured to separate the base and cover layers. Even though the adhesive may be attacked by the volatile material once the heat seal has been ruptured and could lead to loss of the volatile material through the adhesive seal, this will usually be of little detriment since the device will usually be discarded shortly afterwards when the user has smelt the fragrance emitted from the opened device.

### SUMMARY OF THE INVENTION:

Accordingly, the present invention provides a device which comprises a substantially vapour proof envelope containing a fluid material which it is desired to release into the environment by opening of the envelope, the device comprising
a. a base layer having vapour barrier properties, preferably it is substantially vapour proof, which base layer has applied directly to a selected area of a first face thereof a fluid material containing a volatile liquid;
b. a cover layer having vapour barrier properties, preferably it is substantially vapour proof, which cover layer is applied, preferably directly and without an intermediate layer, over said selected area of the said first face of the base layer;
c. a seal between at least the periphery of said base and cover layers, said seal comprising a first component which is re-formable upon replacement of said cover layer so as to prevent further release of said volatile material when the cover layer is replaced upon said base layer, characterised in that the device comprises a second heat seal component, whereby there is formed a substantially vapour proof enclosure for said volatile liquid, said two components of said seal being separable whereby a user can separate at least part of said cover layer from said base layer so as to expose at least part of said first face of said base layer directly to the atmosphere and allow the volatile material carried by the base layer to be released from the base layer by volatilisation.

For convenience,
the term liquid volatile material will be used to identify the material which it is desired to release from the base and/or cover layers by volatilization when the areas of those layers to which the liquid volatile material has be applied are exposed by separation of the base and cover layers; and the term fluid composition will be used herein to denote the fluid composition in which the volatile liquid is applied to the base and/or cover layers, and such fluid compositions may contain other, non-volatile, ingredients such as thickeners for the liquid volatile material and such non-volatile materials may remain upon the surface of the base and/or cover layer once the liquid volatile material has been released from the device.

The device of the invention is of essentially the same construction, operation and application as the device described in our US Patent No 5,439,172, except for the provision of the second heat seal component of the seal, and the subject matter of that patent is incorporated herein by reference. Thus, the device of the invention is preferably a generally planar sampler for a fine fragrance but may be used for the release of other volatile materials, for example aromatic oils in aromatherapy or to release vapours of a medicament, pesticides for use in a glasshouse, rust inhibitors for use in a tool chest, scents of foodstuffs and so one. The volatile liquid is preferably a fine fragrance in the form of a natural or synthetic fragrance oil or an alcohol based solution of the essential fragrance oil as are commercially available. For convenience, the invention will be described in terms of this preferred volatile liquid.

The liquid volatile material may be applied as such to the base and cover layers, for example it may consist essentially of a natural or synthetic fragrance oil diluted with ethanol. However, it may be desired to incorporate a viscosity modifying component into the fragrance oil/ethanol mixture to adjust the viscosity of the mixture so as to render it suitable for application to the base layer using a printing technique as described below. Suitable viscosity modifying agents include fumed silica, cellulosic derivatives, hydrocarbon resins or acrylic or vinylic polymers as are conventionally used as thickeners in the pharmaceutical or cosmetic industries. If desired, the surface adhesive properties of such polymers may be modified to reduce adhesion of the fluid composition containing them to the cover layer making it difficult to peel back the cover layer from the base layer. For example, a polyvinyl alcohol type of thickening agent can be modified by inserting long chain, for example stearic and/or oleic, groups as side branches to the main polyvinyl backbone molecule of the polyvinyl alcohol. Alternatively, the surface of the cover layer to be in contact with that area of the base layer carrying the fluid composition may be treated with a dermatologically acceptable abhesive which is substantially free from odours, for example stearic or oleic acid or an ester thereof.

It is preferred that the base and cover layers be made from a thermoplastic heat sealable polymer which is micropermeable to the volatile liquid, notably a polyester sheet or a laminated or composite material which has a polyester face or layer to which the fluid material containing the liquid volatile material is applied. In some cases it may be desirable to provide a layer of a polyvinylidene dichloride or other substantially vapour proof polymer on the exposed faces of the base and/or cover layer to further minimise loss of volatile material from the device. If desired, the cover layer may be made from such a vapour barrier polymer where the baser layer absorbs the liquid volatile material sufficiently to act as the sole or major reservoir for the volatile liquid.

The face of the base and/or cover layer to which the fluid material is to be applied may have been subjected to a pre-treatment, for example a corona discharge treatment or pre-coating with a polyacrylate polymer as known in the art to enhance the wetting of the surface of the layer by the fluid material applied thereto. This assists the migration of the volatile liquid into the polymer matrix of the layer.

Such a sampler is preferably made by applying the fragrance oil to the base layer using a roller applicator as if the fragrance were a printers ink. Preferably, the fragrance is applied using a flexographic or offset lithographic printing process in which the fragrance oil, or an ethanol dilution thereof, optionally containing viscosity modifying agents to achieve the optimum viscosity for the particular printing machine being used and the desired loading of fluid material to be applied, is used in place of the ink normally used in such printing processes. A particularly preferred process for making the devices of the present invention involves making the device using the techniques described in our US Patent No 5 439 172, notably those set out in claim 10 thereof and subsequently forming the heat seal radially inwardly or outwardly of the adhesive seal using conventional heat sealing techniques and equipment.

The sampler finds particular use as an insert into a magazine or the like, either by direct application to a page of the magazine or by application to a printed insert sheet which is then interleaved into the magazine. It will therefore usually be preferred to apply a coating or layer of a pressure sensitive adhesive to that face of the base layer not carrying the volatile material and a slip sheet, for example a siliconised paper layer, over that adhesive layer using conventional techniques.

For convenience, the invention will be described hereinafter in terms of a planar fragrance sampler of the above type.

The first, reformable, component of the seal between the base and cover layers of the device of the invention can be provided by the autoadhesive properties of the base and cover layers. However, it is preferred to provide this component by means of continuous or discontinuous annulus of a pressure sensitive adhesive applied to the base and/or cover layer around the area to which the material containing the volatile liquid has been applied. Such an adhesive annulus can be applied to the base and/or cover layer using any appropriate technique, for example using a printing type of roller applicator as described in our US Patent 5,439,172. Suitable adhesives are of the polyacrylate of polyacrylic acid type.

The second heat seal component is typically of the same plan shape as the first component, for example as a smaller or larger diameter ring seal substantially concentric with the adhesive seal. The heat seal can be located radially inwardly and/or outwardly of the adhesive seal. In the first case, the heat seal minimises contact between the adhesive and the fragrance; in the second case the heat seal does not prevent lateral migration of the fragrance to the adhesive, but minimises any further lateral migration of the fragrance. If desired, heat seals can be formed both radially inward and radially outward of the adhesive annulus to obtain both benefits.

The optimal position for the heat seal will depend upon a number of factors, for example the expected storage period and the forms of plastic which are used to make the base and cover layers. In general, unless strength considerations prevent it, it is preferred to form the heat seal immediately radially adjacent, ie. contiguous with or within 2 mms radially, of the adhesive seal. If desired, the heat seal can be located within the radial extent of the adhesive seal, for example where heat aids curing of the adhesive seal. Furthermore, the pressure required to form the heat seal may assist formation of a good bond between the adhesive of the adhesive seal and the base and/or cover layers. For convenience, the invention will be described in terms of an annular heat seal formed radially inwardly of an annular adhesive seal.

The annular adhesive seal is preferably formed first by applying a suitable adhesive, for example a water based acrylic adhesive, to the base layer and the applying the cover layer to the base layer to form an initial envelope for the fluid volatile material which has already been applied to the base layer. The heat seal is then formed by applying a suitable heated sealer bar under pressure to the initial envelope to cause partial fusion of the base and cover layers. A conventional reciprocating sealer bar mechanism may be used to apply heat and pressure to the envelope. The heat necessary to cause fusion of the base and cover layers can be achieved using hot air, infra red, electrical resistance or induction heating or by any other suitable means.

However, it is preferred to use a moving belt type heat sealer in which at least part of the sealer mechanism travels with the moving web of the base and cover layers so that the duration of the pressure and heat cycles can be comparatively prolonged without interrupting the flow of the web. In this way the sealing process can be optimised without significantly slowing down the production of the samplers using conventional printing processes. Typically, a moving belt heat sealer comprises a static support member, eg. a plate of polished stainless steel, over which the web of material passes. Due to the initial seal formed by the adhesive seal, the webs of the base and cover layers travel together as a single web. However, if desired, guide rails or rollers can be used to assist free flow of the web through the heat sealing station and to minimise the risk of separation of the webs. Typically, the static support member is a substantially horizontal member which underlies the path of the web. However, other orientations may be used if desired.

The moving component of the moving belt type heat sealer can take the form of a heated metal belt, for example one which has apertures therein which is heated by means of a hot air blower which also partially fuses the material exposed through the apertures in the belt. The belt can be supported on a static anvil or the like which bears against the belt to apply pressure to the belt and hence to the material passing through the nip between the moving belt and the static lower support member. The anvil can be spring loaded to apply the desired pressure. The belt is provided with raised ribs or the like which define the shape of the seal to be formed.

Alternatively, the moving belt can be supported by a rotating drum or roller which carries the necessary raised rib to form the seal, or the belt can be provided by the surface of the rotating drum or roller.

The speed of travel of the belt and/or the rotation of the roller or drum can be selected over a wide range depending upon the temperature achieved by the heating means and the heal sealing characteristics of the polymer from which the base and cover layers are formed. It will thus usually be possible to achieve the temperatures conditions and pressure dwell time required for adequate heat sealing of the base and cover layers without significant loss in the linear through put of the web being heat sealed.

If desired, accumulator loops of the web can be created upstream and/or downstream of the heat sealing station to accommodate fluctuations in the linear flow of the web to or from the heat sealing station, whilst preserving a substantially uniform rate of travel of the web through the heat sealing station.

The heat seal can be formed as a continuous annular seal. However, it is within the scope of the present invention to form the seal using a discontinuous heater/sealer bar so that a heat seal having intermittent areas of weakness in the heat seal formed can be achieved. Such a heat seal will be more readily separated than one formed using a continuous heat sealer bar. Surprisingly, we have found that it is possible to select the seal forming conditions of heat and pressure and the gap between adjacent sections of the heat sealer bar so that the resultant seal spreads laterally around to area of direct action of the heat sealer bar to achieve overlapping areas of weakly sealed material between areas where a full heat seal has been formed.

It is particularly preferred to form the heat seal after the adhesive ring seal has been formed between the base and cover layers, since this initial adhesive seal can be formed using printing techniques so that accurate registration between the area to which the fluid material has been applied and the surrounding adhesive ring can be readily achieved using conventional printing equipment and techniques. The adherent base and cover layers then travel and a unitary body with reduced risk of relative movement between the two layers before the heat seal is formed.

The device may be provided with other features which enhance its utility. Thus, for example a layer of adhesive can be applied to the face of the base layer to which the fluid material has not been applied so that the device can be attached to a user's clothing or other surface. This adhesive layer can be a pressure sensitive adhesive of the type used to form the adhesive strip(s) between the base and cover layers of the device. The adhesive layer may be protected by a removable layer, for example a siliconised paper sheet, which is removed prior to use.

In use, a user separates the cover layer from the base layer by rupturing the heat seal and separating the adhesive seal. If desired, a pull tab may be formed at the periphery of the cover layer which is not adhered to the base layer to provide a pull tab by which the user can peel back the cover payer. Having exposed the area of the base layer to which the fluid material has been applied, the user can then either sniff the fragrance released from the exposed base layer, or can wipe the exposed surface of the base layer against his or her skin so as to transfer some of the fluid material to his or her skin. Alternatively, the user can secure the opened device to an area of his or her clothing using the adhesive layer applied to the other face of the base layer to which the fluid material has been applied. Once the user has sampled the fragrance to his or her satisfaction, the cover layer can be replaced upon the base layer and is secured in position by the adhesive annulus. The device thus reforms a sealed envelope which prevents further release of the volatile material until the cover payer is removed again. Preferably, the heat seal is formed so that the user does not wholly separate the cover layer from the base layer, facilitating reapplication of the cover layer to the base layer. For example, the areas of weakness in the heat seal can be formed over only part of the circumference of the heat seal so that part of the heat seal is more resistant to rupture than the weakened area and forms a permanent attachment of the cover layer to the base layer.

### DESCRIPTION OF THE DRAWINGS:

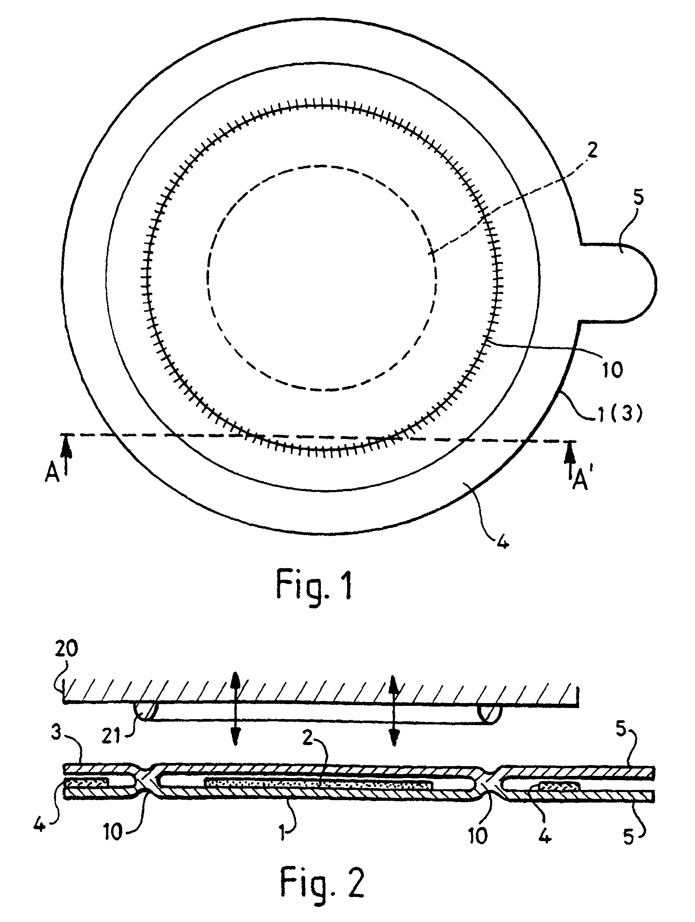

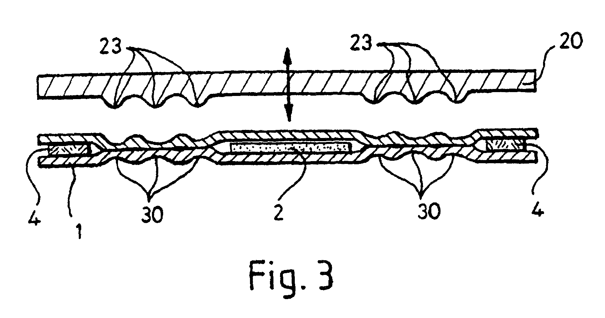

A preferred form of the device of the invention will now be described by way of illustration with respect to the accompanying drawings in which Figure 1 is a plan view of the device from above; Figure 2 is a vertical section through the device of Figure 1 along the diameter of the device; and Figure 3 is a detailed vertical section along the line A-A' of part of an alternative form of the heat seal of the device of Figure 1 with part of the heater/sealer bar used to form the heat seal shown diagrammatically.

### DESCRIPTION OF THE PREFERRED EMBODIMENT:

The device comprises a 20 micrometre thick base layer 1 of a polyester polymer, such as that sold under the Registered Trade Mark MYLAR. A coating 2 of a fragrance oil/ethanol mixture is applied by a conventional roller printer to the central area of the base layer. A cover layer 3 of the same MYLAR material as the base layer 1 is secured to the base layer by an annular ring 4 of a pressure sensitive water based acrylic adhesive. The cover layer 3 and/or the base layer 1 has a radially extending pull tab 5 whereby the cover layer can be peeled back from the base layer 1 to expose the fragrance coated area 2 of the base layer to release the volatile fragrance.

Such a device can be prepared by the technique described in relation to Figures 3 and 4 of our US Patent No 5 439 172.

A second seal 10 is formed radially inward of the adhesive annulus 4 using a conventional reciprocating heat sealer bar 20. This bar may carry a continuous raised annular rib 21 to form a continuous annular heat seal as shown in Figure 2, requiring substantially the same force throughout its circumference to separate the heat seal 10. However, as shown in Figure 3, the bar 20 can carry a plurality of rounded projections or pimples 23 formed as an annular grouping. Such pimples apply localised heat and pressure to the cover and base layers 1 and 3 to form localised heat seals 30 between the two layers. Such seals 30 have a central portion where a full strength heat seal is formed. However, the strength of the heat seal reduces with increasing distance from the central portion due to the reduction in pressure applied to the layers by the radially outer portions of the pimples. By suitable spacing between the pimples 23, the localised heat seals overlap one another to form an overall heat seal having areas of weakness within the overall seal. Such a heat seal can be more readily separated than one formed using a continuous heat sealer as in Figure 2. Such a weaker area of the heat seal may be formed uniformly around the annular heat seal 10. However, it may be preferred to form such a weakened area over only part of the circumference of the heat seal, for example over an arc extending for from 300 to 350° of the circumference, with the remainder of the seal formed as a full heat seal. Thus, the base and cover layers can be readily separated over the weakened area, but a separation resistant area is left over the full heat seal area. Such a construction reduces the risk that the cover layer is wholly removed from the base layer and the residual heat sealed area provides a means for retaining registration of the cover layer with the base layer when the cover layer is re-applied to the base layer.

The heat seal 10 can be formed using conventional reciprocating bar or moving belt heat sealing techniques and equipment and provides a surprisingly effective barrier to lateral migration of the fragrance oil into the adhesive ring 4. The formation of the heat seal can be combined with the operation of severing the device from the web of sheets of plastic from which it is formed, notably where the heat seal is radially outward of the adhesive seal.

## Claims

1. A device which comprises a substantially vapour proof envelope containing a fluid material which it is desired to release into the environment by opening of the envelope, the device comprising
a. a base layer having vapour barrier properties, which base layer has applied directly to a selected area of a first face thereof a fluid material containing a volatile liquid;
b. a cover layer having vapour barrier properties, which cover layer is applied over said selected area of the said first face of the base layer;
c. a seal between at least the periphery of said base and cover layers, said seal comprising a first component which is re-formable upon replacement of said cover layer so as to prevent further release of said volatile material when the cover layer is replaced upon said base layer, **characterised in that** the device comprises a second heat seal component, whereby there is formed a substantially vapour proof enclosure for said volatile liquid, said two components of said seal being separable whereby a user can separate at least part of said cover layer from said base layer so as to expose at least part of said first face of said base layer directly to the atmosphere and allow the volatile material carried by the base layer to be released from the base layer by volatilisation.

2. A device as claimed in claim 1, **characterised in that** the base layer is made from a material having at least a surface layer made from a polymer which is micropermeable to the liquid volatile material.

3. A device as claimed in either of claims 1 or 2, **characterised in that** the first component of said seal is an adhesive seal.

4. A device as claimed in any one of the preceding claims, **characterised in that** the heat seal is located radially inwardly of said first component of said seal.

5. A device as claimed in any one of the preceding claims, **characterised in that** the heat seal component of the seal if formed with areas of weakness extending over at least part of the circumference of the seal.

6. A device as claimed in claim 5, **characterised in that** at least part of the circumferential extent of the heat seal is formed without areas of weakness.

7. A device as claimed in any one of claims 1 to 3 and 5 to 6, **characterised in that** the heat seal component of the seal lies radially within the area of the first component of the seal.

## Patentansprüche

1. Einrichtung, die eine im wesentlichen dampfundurchlässige Hülle umfaßt, die ein flüssiges Material enthält, das in die Umgebung freigesetzt werden soll, indem die Hülle geöffnet wird, wobei die Einrichtung folgendes umfaßt:
a. eine Basisschicht mit Dampfsperreigenschaften, auf eine erste Seite derer in einem ausgewählten Bereich ein eine flüchtige Flüssigkeit enthaltendes flüssiges Material direkt aufgetragen ist;
b. eine Deckschicht mit Dampfsperreigenschaften, die auf die erste Seite der Basisschicht in dem ausgewählten Bereich aufgetragen ist;
c. ein Siegel mindestens zwischen der Peripherie der Basisschicht und der Deckschicht, wobei das Siegel eine erste Komponente enthält, die, wenn die Deckschicht wieder an der alten Stelle angeordnet wird, zurückgebildet werden kann, um die weitere Freisetzung des flüchtigen Materials zu verhindern, wenn die Deckschicht wieder auf der Basisschicht angeordnet wird, **dadurch gekennzeichnet, daß** die Einrichtung eine zweite Heißsiegelkomponente umfaßt, wodurch für die flüchtige Flüssigkeit eine im wesentlichen dampfundurchlässige Umhüllung ausgebildet wird, wobei die beiden Komponenten des Siegels getrennt werden können, wodurch ein Benutzer mindestens einen Teil der Deckschicht von der Basisschicht trennen kann, damit mindestens ein Teil der ersten Seite der Basisschicht direkt zur Atmosphäre freigelegt wird und das von der Basisschicht getragene flüchtige Material von der Basisschicht durch Verdampfung freigesetzt werden kann.

2. Einrichtung nach Anspruch 1, **dadurch gekennzeichnet, daß** die Basisschicht aus einem Material hergestellt ist, das mindestens eine aus einem Polymer hergestellte Oberflächenschicht aufweist, die für das flüssige flüchtige Material mikropermeabel ist.

3. Einrichtung nach einem der Ansprüche 1 oder 2, **dadurch gekennzeichnet, daß** die erste Komponente des Siegels ein Klebesiegel ist.

4. Einrichtung nach einem der vorhergehenden Ansprüche, **dadurch gekennzeichnet, daß** das Heißsiegel bezüglich der ersten Komponente des Siegels radial nach innen angeordnet ist.

5. Einrichtung nach einem der vorhergehenden Ansprüche, **dadurch gekennzeichnet, daß** die Heißsiegelkomponente des Siegels mit schwachen Bereichen ausgebildet ist, die sich über mindestens einen Teil des Umfangs des Siegels erstrecken.

6. Einrichtung nach Anspruch 5, **dadurch gekennzeichnet, daß** mindestens ein Teil der umfangsmäßigen Erstreckung des Heißsiegels ohne schwache Bereiche ausgebildet ist.

7. Einrichtung nach einem der Ansprüche 1 bis 3 und 5 und 6, **dadurch gekennzeichnet, daß** die Heißsiegelkomponente des Siegels radial innerhalb des Bereichs der ersten Komponente des Siegels liegt.

## Revendications

1. Dispositif qui comprend une enveloppe sensiblement étanche à la vapeur contenant un matériau fluide qui est destiné à être libéré dans l'environnement en ouvrant l'enveloppe, le dispositif comprenant :
a - une couche de base ayant des propriétés d'écran d'étanchéité à la vapeur, un matériau fluide contenant un liquide volatile ayant été appliqué directement sur une surface sélectionnée d'une première face de cette couche de base ;
b - une couche de revêtement ayant des propriétés d'écran d'étanchéité à la vapeur, cette couche de revêtement étant appliquée sur ladite surface sélectionnée de ladite première face de la couche de base ;
c - un joint entre au moins la périphérie desdites couches de base et de revêtement, ledit joint comprenant un premier composant qui peut être reformé par remplacement ladite couche de revêtement de sorte à empêcher une libération supplémentaire dudit matériau volatile lorsque la couche de revêtement est remplacée sur ladite couche de base, **caractérisé en ce que** le dispositif comprend un second composant de joint thermique, moyennant quoi on forme une enveloppe sensiblement étanche à la vapeur pour ledit liquide volatile, lesdits deux composants dudit joint étant séparables moyennant quoi un utilisateur peut séparer au moins une partie de ladite couche de revêtement de ladite couche de base de sorte à exposer au moins une partie de ladite première face de ladite couche de base directement à l'atmosphère et permettre au matériau volatile porté par la couche de base d'être libéré de la couche de base par volatilisation.

2. Dispositif selon la revendication 1, **caractérisé en ce que** la couche de base est réalisée à partir d'un matériau ayant au moins une couche de surface réalisée à partir d'un polymère qui est micro-perméable au matériau liquide volatile.

3. Dispositif selon l'une des revendications 1 ou 2, **caractérisé en ce que** le premier composant dudit joint est un joint adhésif.

4. Dispositif selon l'une quelconque des revendications précédentes, **caractérisé en ce que** le joint thermique est situé de façon radiale à l'intérieur dudit premier composant dudit joint.

5. Dispositif selon l'une quelconque des revendications précédentes, **caractérisé en ce que** le composant de joint thermique du joint est formé avec des surfaces de faiblesse s'étendant sur au moins une partie de la circonférence du joint.

6. Dispositif selon la revendication 5, **caractérisé en ce qu'**au moins une partie de l'étendue circonférentielle du joint thermique est formée sans surfaces de faiblesse.

7. Dispositif selon l'une quelconque des revendications 1 à 3 et 5 à 6, **caractérisé en ce que** le composant de joint thermique du joint est disposé de façon radiale à l'intérieur de la surface du premier composant du joint.
